(19) Europäisches Patentamt
European Patent Office
Office européen des brevets

(11) Veröffentlichungsnummer: **0 243 668**
**A2**

(12) # EUROPÄISCHE PATENTANMELDUNG

(21) Anmeldenummer: **87104214.9**

(22) Anmeldetag: **21.03.87**

(51) Int. Cl.⁴: **C07C 103/76** , A01N 37/18 , A01N 37/50

(30) Priorität: **04.04.86 DE 3611193**

(43) Veröffentlichungstag der Anmeldung: **04.11.87 Patentblatt 87/45**

(84) Benannte Vertragsstaaten: **AT BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Tarnow, Horst, Dr.**
**Carl-Diem-Weg 6**
**D-4018 Langenfeld(DE)**
Erfinder: **Homeyer, Bernhard, Dr.**
**Obere Strasse 28**
**D-5090 Leverkusen 3(DE)**

(54) **N-Substituierte Benzamide.**

(57) Es werden neue N-substituierte Benzamide der Formel (I) bereitgestellt,

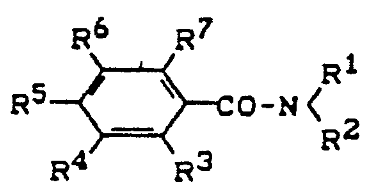

(I)

in welcher
$R^1$ für Wasserstoff oder Alkyl steht,
$R^2$ für Halogenalkyl mit mehr als 1 Kohlenstoffatom oder für durch Halogen oder Halogenalkyl substituiertes Cycloalkyl steht,
$R^3$, $R^4$, $R^5$, $R^6$ und $R^7$ gleich oder verschieden sind und für Wasserstoff, Halogen, Nitro, Alkyl, Halogenalkyl, Halogenalkoxy, Halogenalkylthio oder für den Rest -CONR$^1$R$^2$ stehen.

Die neuen Benzamide (I) besitzen ausgezeichnete pestizide und insbesondere insektizide und nematizide Eigenschaften.

EP 0 243 668 A2

## N-Substituierte Benzamide

Die Erfindung betrifft neue N-substituierte Benzamide, ein Verfahren zu ihrer Herstellung und ihre Verwendung in Schädlingsbekämpfungsmitteln, insbesondere als Insektizide und Nematizide.

Es ist bekannt, daß bestimmte Phosphorsäureesteramide wie z.B. O-Ethyl-O-(3-methyl-4-methylthiophenyl)-N-isopropyl-phosphorsäureesteramid (Freibezeichnung: Fenamiphos) sehr gute insektizide und nematizide Eigenschaften aufweisen (vergl. US-PS 2 978 479 sowie Pesticide Manual herausgegeben von British Crop Protection Council, 5. Auflage (1977), Seite 262 mit einer Beschreibung des Handelsproduktes Fenamiphos).

Es wurden nun neue N-substituierte Benzamide der Formel (I) gefunden,

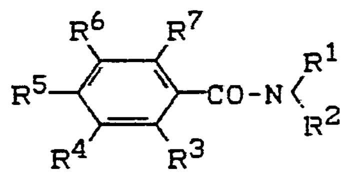

(I)

in welcher

$R^1$ für Wasserstoff oder Alkyl steht,

$R^2$ für Halogenalkyl mit mehr als einem Kohlenstoffatom oder für durch Halogen oder Halogenalkyl substituiertes Cycloalkyl steht,

$R^3$, $R^4$, $R^5$, $R^6$ und $R^7$ gleich oder verschieden sind und für Wasserstoff, Halogen, Nitro, Alkyl, Halogenalkyl, Halogenalkoxy, Halogenalkylthio oder für den Rest -CONR$^1$R$^2$ stehen.

Weiterhin wurde gefunden, daß man die neuen N-substituierten Benzamide der Formel (I) erhält, wenn man Benzoylhalogenide der Formel (II)

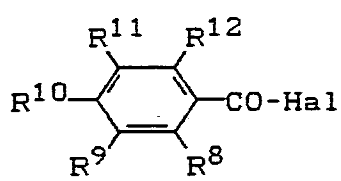

(II)

in welcher

$R^8$, $R^9$, $R^{10}$, $R^{11}$ und $R^{12}$ gleich oder verschieden sind und für Wasserstoff, Halogen, Nitro, Alkyl, Halogenalkyl, Halogenalkoxy, Halogenalkylthio oder für den Rest -CO-Hal stehen und

Hal für Halogen steht,

mit Aminen der Formel (III)

$HNR^1R^2$ (III)

in welcher

$R^1$ und $R^2$ die oben angegebenen Bedeutungen haben,

bzw. den entsprechenden Hydrohalogeniden, gegebenenfalls in Gegenwert von Säureakzeptoren und gegebenenfalls in Gegenwart von Verdünnungsmitteln umsetzt.

Die neuen N-substituierten Benzamide der Formel (I) zeichnen sich durch eine sehr gute nematizide und insektizide Wirkung aus.

Die Erfindung betrifft vorzugsweise Verbindungen der Formel (I), in welcher

$R^1$ für Wasserstoff oder Alkyl mit 1 bis 6 Kohlenstoffatomen steht,

$R^2$ für Halogenalkyl mit 2 bis 8 Kohlenstoffatomen und 1 bis 12 Halogenatomen wie Fluor und/oder Chlor, sowie für durch Halogen wie Fluor und/oder Chlor oder Halogen-$C_1$-$C_2$-alkyl substituiertes Cycloalkyl mit 3 bis 8 Kohlenstoffatomen steht,

$R^3$, $R^4$, $R^5$, $R^6$ und $R^7$ gleich oder verschieden sind und für Wasserstoff, Fluor, Chlor, Brom, Nitro, Alkyl mit 1 bis 4 Kohlenstoffatomen, Halogenalkyl, Halogenalkoxy oder Halogenalkylthio mit jeweils 1 bis 4 Kohlenstoffatomen im Alkylteil und 1 bis 6 Halogenatomen wie Fluor und/oder Chlor sowie für den Rest -CONR$^1$R$^2$ stehen.

Besonders bevorzugt sind die neuen N-substituierten Benzamide der Formel (I), in welcher
$R^1$ für Wasserstoff oder Alkyl mit 1 bis 4 Kohlenstoffatomen steht,
$R^2$ für Halogenalkyl mit 2 bis 6 Kohlenstoffatomen und 1 bis 8 Fluor-oder 1 bis 8 Fluor-und Chloratomen sowie für durch Fluor, Chlor und/oder Trifluormethyl substituiertes Cycloalkyl mit 3 bis 6 Kohlenstoffatomen steht,
$R^3$, $R^4$, $R^5$, $R^6$ und $R^7$ gleich oder verschieden sind und für Wasserstoff, Fluor, Chlor, Brom, Nitro, Alkyl mit 1 oder 2 Kohlenstoffatomen, Halogenalkyl, Halogenalkoxy oder Halogenalkylthio mit jeweils 1 oder 2 Kohlenstoffatomen im Alkylteil und 1 bis 4 Fluor-und/oder Chloratomen sowie für den Rest -CONR$^1$R$^2$ stehen.

Ganz besonders bevorzugt sind die neuen Verbindungen der Formel (I), in welcher
$R^1$ für Wasserstoff, Methyl, Ethyl, n-Propyl, i-Propyl oder n-Butyl steht,
$R^2$ für 2-Fluorethyl, 2,2-Difluorethyl, 2,2,2-Trifluorethyl, 3-Fluor-prop-1-yl, 3,3-Difluor-prop-1-yl, 3,3,3-Trifluor-prop-1-yl, 3-Fluor-prop-2-yl, 3,3-Difluor-prop-2-yl, 3,3,3-Trifluor-prop-2-yl, 2-Fluor-2-methyl-prop-2-yl, 2,2-Difluor-2-methyl-prop-2-yl, 2-Methyl-2,2,2-trifluor-prop-2-yl, 2-Methyl-3,3,3-trifluor-prop-1-yl, 2-Methyl-3,3,3-chlordifluor-prop-1-yl, 2-Methyl-3,3,3-dichlorfluor-prop-1-yl, 1,3-Difluor-prop-2-yl, 1,1,1,3,3,3-Hexafluor-prop-2-yl, 3-Fluor-2-fluormethyl-prop-2-yl, 1,3-Difluor-2-fluormethyl-prop-2-yl, 1-Trifluormethyl-cycloprop-1-yl und 2,2-Difluor-cycloprop-1-yl steht und
$R^3$, $R^4$, $R^5$, $R^6$ und $R^7$ gleich oder verschieden sind und für Wasserstoff, Fluor, Chlor, Brom, Nitro, Methyl, Trifluorme thyl, Trifluormethoxy, Trifluormethylthio sowie für den Rest -CONR$^1$R$^2$ stehen.

Verwendet man beispielsweise für das erfindungsgemäße Verfahren 2,6-Difluorbenzoylchlorid und 2-Amino-3,3,3-trifluorpropan als Ausgangsstoffe, so kann die entsprechende Reaktion durch das folgende Formelschema skizziert werden:

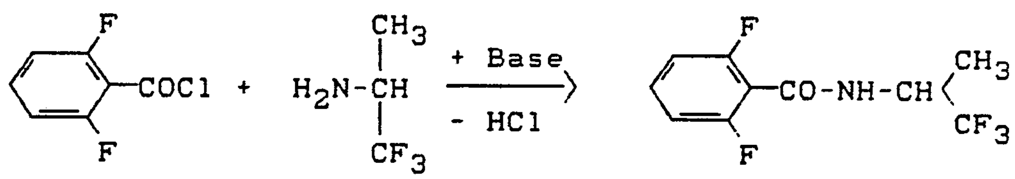


Verwendet man beispielsweise für das erfindungsgemäße Verfahren o-Phthalsäuredichlorid und 1-Amino-3,3,3-trifluorpropan als Ausgangsstoffe, so kann die entsprechende Reaktion durch das folgende Formelschema skizziert werden:

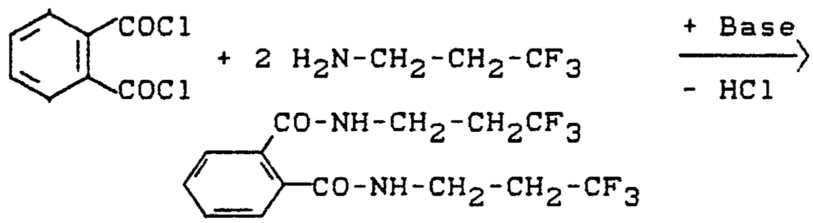


Die beim erfindungsgemäßen Verfahren als Ausgangsstoffe zu verwendenden Benzoylhalogenide sind durch die Formel (II) allgemein definiert. In dieser Formel stehen $R^8$, $R^9$, $R^{10}$, $R^{11}$ und $R^{12}$ vorzugsweise für Wasserstoff, Fluor, Chlor, Brom, Nitro, $C_1$-$C_4$-Alkyl, Halogen-$C_1$-$C_4$-alkyl, Halogen-$C_1$-$C_4$-alkoxy und Halogen-$C_1$-$C_4$-alkylthio. Hal steht für Halogen wie insbesondere für Fluor oder Chlor.

Die Verbindungen der Formel (II) sind bekannt und/oder lassen sich nach bekannten Methoden herstellen. Siehe dazu beispielsweise DE-AS 2 123 236, DE-OS 2 601 780, DE-OS 2 637 947, DE-OS 3 217 619, DE-OS 3 217 620 und EP-OS 122 449.

Als Beispiele für die Verbindungen der Formel (II) seien genannt:

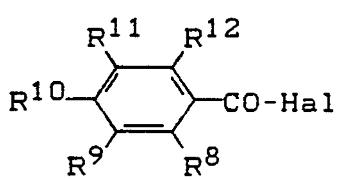 (II)

Hal = Chlor oder Fluor

Tabelle 1

| $R^8$ | $R^9$ | $R^{10}$ | $R^{11}$ | $R^{12}$ |
|---|---|---|---|---|
| F | H | H | H | F |
| H | H | H | H | H |
| H | H | H | H | F |
| H | H | H | F | H |
| H | H | F | H | H |

Tabelle 1 - Fortsetzung

| $R^8$ | $R^9$ | $R^{10}$ | $R^{11}$ | $R^{12}$ |
|---|---|---|---|---|
| H | H | F | H | F |
| F | H | F | H | F |
| F | F | F | F | F |
| H | F | Cl | H | Cl |
| F | Cl | F | Cl | F |
| H | H | H | H | Cl |
| H | H | H | Cl | H |
| H | H | Cl | H | H |
| Cl | H | H | H | Cl |
| H | Cl | H | H | Cl |
| H | H | Cl | H | Cl |
| H | H | H | H | $CF_3$ |
| H | H | H | $CF_3$ | H |

Tabelle 1 - Fortsetzung

| $R^8$ | $R^9$ | $R^{10}$ | $R^{11}$ | $R^{12}$ |
|---|---|---|---|---|
| H | H | $CF_3$ | H | H |
| H | H | H | H | $CH_3$ |
| H | H | H | $CH_3$ | H |
| H | H | $CH_3$ | H | H |
| H | H | $NO_2$ | H | H |
| H | H | H | $NO_2$ | H |
| H | H | Cl | $NO_2$ | H |
| H | H | H | H | COCl |
| H | H | COCl | H | H |
| H | H | H | H | COF |
| H | H | COF | H | H |

Die beim erfindungsgemäßen Verfahren außerdem als Ausgangsstoffe zu verwendenden Amine und die entsprechenden Hydrohalogenide sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) vorzugsweise für diese Substituenten genannt wurden. Als Hydrohalogenide werden bevorzugt die Hydrochloride oder die Hydrobromide eingesetzt.

Als Beispiele für die Verbindungen der Formel (III) seien genannt:

$HNR^1R^2$ (III)

Tabelle 2

| $R^1$ | $R^2$ | $R^1$ | $R^2$ |
|---|---|---|---|
| H | $-CH_2-CHF_2$ | H | $-CH_2-CF_3$ |
| H | $-CF_2-CF_3$ | H | $-CH_2-CF_2-CH_3$ |
| H | $-CH_2-CH_2-CHF_2$ | H | $-CH_2-CH_2-CHClF$ |
| H | $-CH-CH_2-CH_2-CH_3$<br>\|<br>$CF_3$ | H | $-CH-CH_2-CH_2-CF_3$<br>\|<br>$CF_3$ |
| H | $-CH-CH(CH_3)_2$<br>\|<br>$CF_3$ | H | $-CH_2-CH_2-CF_3$ |
| H | $-CH_2-CH_2-CClF_2$ | H | $-CH_2-CH_2-CCl_2F$ |

Tabelle 2 - Fortsetzung

| $R^1$ | $R^2$ | $R^1$ | $R^2$ |
|---|---|---|---|
| H | $-CH_2-CHF-CF_3$ | H | $-CH_2-CF_2-CF_3$ |
| H | $-CH_2-CH_2-CH_2-CF_3$ | H | 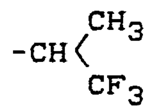 |
| H | $-C(CH_3)_2-CH_2F$ | H | $-CH(CF_3)_2$ |
| H | $-C(CH_3)_2CF_3$ | H | 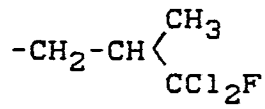 |
| H | 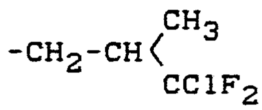 | H | 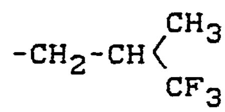 |
| H | 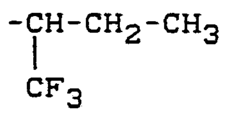 | H | $-CH_2-CH_2F$ |
| H | $-CH_2-CH_2-CH_2F$ | H | 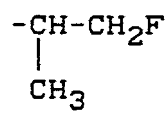 |
| H | 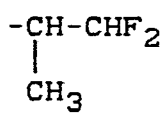 | H | $-C(CH_3)_2-CHF_2$ |
| H | 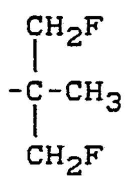 | H | $-C(CH_2F)_3$ |
| H | 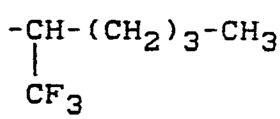 | H | 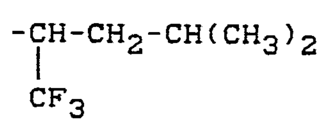 |
| H | 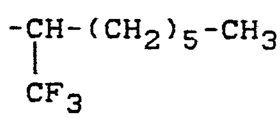 | $-CH_3$ | $-CH_2-CF_3$ |

Tabelle 2 - Fortsetzung

| $R^1$ | $R^2$ | $R^1$ | $R^2$ |
|---|---|---|---|
| $-CH_3$ | $-CH_2-CH_2-CF_3$ | $-C_2H_5$ | $-CH_2-CF_3$ |
| $-C_3H_7-n$ | $-CH_2-CF_3$ | $-C_4H_9-n$ | $-CH_2-CF_3$ |
| H | $-CH(CH_2F)_2$ | H | 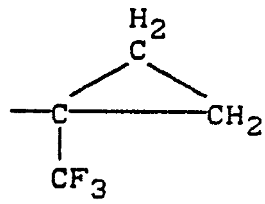 |
| H | 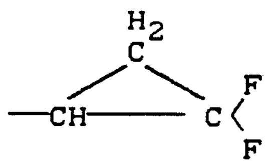 | | |

und die entsprechenden Hydrochloride bzw. -bromide der oben genannten Verbindungen der Formel (III).

Die Verbindungen der Formel (III) und die entsprechenden Hydrohalogenide sind zum Teil bekannt (vergl. z. B. J. Org. Chem. 24 , 1256 - 1259 (1969); J. Org. Chem. 27, 1406 - 1409 (1962); Isvest. Akad. Nauk. SSSR. Ser. Khim. 1966, 226 - 228; Isvest. Akad. Nauk. SSSR, Ser. Khim. 1966, 1518 - 1523; J. Med. Chem. 22, 1130 - 1133 (1979); DE-OS 30 18 020; EP-A 39 813; DE-OS 32 18 966; EP-A 73 974; DE-OS 33 07 234; Ind. Eng. Chem. 48, 209 - 213 (1956); J. Am. Chem. Soc. 102, 4958 - 4959 (1980) und die Herstellungsbeispiele).

Unter die Formel (III) fallenden neue Amine werden beispielsweise wie folgt dargestellt.

Neue Amine der Formel

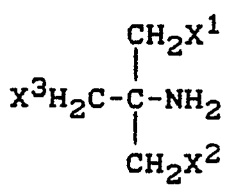 (IV),

in welcher die Reste $X^1$ bis $X^3$ gleich oder verschieden sein können und für Wasserstoff oder Fluor stehen, wobei wenigstens einer der Reste $X^1$ bis $X^3$ für Fluor steht,
indem man bei der Herstellungsvariante A)

(a) die halogenierten Pivalsäurehalogenide der allgemeinen Formel (V)

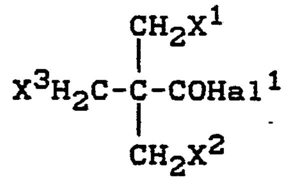

(V),

in welcher X' bis X³ die oben angegebene Bedeutung haben und Hal' für Fluor oder Chlor steht, mit einem Azidgruppen übertragenden Reagenz in die fluorierten Pivalsäureazide der allgemeinen Formel (VI)

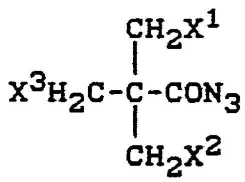

(VI),

in welcher X' bis X³ die oben angegebene Bedeutung haben, umsetzt und

(b) die gegebenenfalls isolierten Verbindungen der allgemeinen Formel (VI) thermisch zu den fluorierten Isocyanaten der allgemeinen Formel (VII)

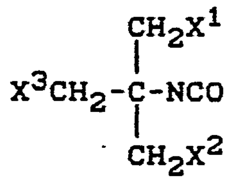

(VII),

in welcher X' bis X³ die oben angegebene Bedeutung haben, zersetzt und

(c) die gegebenenfalls isolierten Verbindungen der allgemeinen Formel (VII) z.B. durch salzsaure Hydrolyse in die fluorierten tertiären Butylamin-Hydrochloride der allgemeinen Formel (VIII)

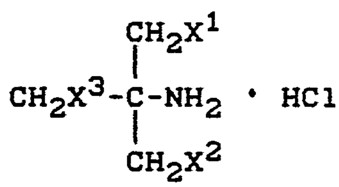

(VIII),

in welcher X' bis X³ die oben angegebene Bedeutung haben, umwandelt und

(d) die gegebenenfalls isolierten Verbindungen der allgemeinen Formel (VIII) durch Behandlung mit Base in die fluorierten tertiären Butylamine der allgemeinen Formel (IV) überführt, oder indem man in der Verfahrensvariante B)

die Isocyanate der Formel (VII)

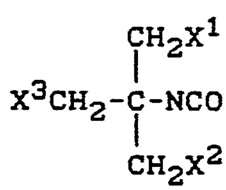

(VII),

in welcher X' bis X³ die oben angegebene Bedeutung haben, durch alkalische Hydrolyse direkt in die fluorierten tertiären Butylamine der allgemeinen Formel (IV) überführt.

oder daß man in der Verfahrensvariante C)
Pivalsäureamide der allgemeinen Formel (IX)

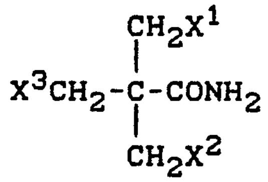

(IX),

in welcher X' biz X³ die oben angegebene Bedeutung haben,
mit Hypohalogenit umsetzt.

Unter die Formel (III) fallenden neue Cyclopropylamine der Formel

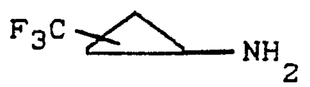

(X)

werden beispielsweise hergestellt, indem man die bekannten Cyclopropylcarbonsäureamine der Formel

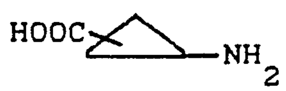

(XI)

mit Schwefeltetrafluorid im wasserfreien Fluorwasserstoff im Autoklaven umsetzt.

Cyclopropancarbonsäureamine der Formel (XI) sind bekannt (siehe z.B. J. Chem. Soc. 1960, 2119-2132, ibid. 1962, 3977-3980; Synthesis 1978, 46; Coll. Czech. Chem. Comm. 47, 2291-2305 (1982)).

Das erfindungsgemäße Verfahren zur Herstellung der neuen N-substituierten Benzamide der Formel (I) wird vorzugsweise unter Verwendung von Verdünnungsmitteln durchgeführt. Als Verdünnungsmittel kommen dabei Wasser und praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium-und Kaliumhydroxid, Erdalkalihydroxide wie z. B. Calciumhydroxid, Alkalicarbonate und -alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Chinolin, 1,5-Diazabicyclo-[4,3,0]-non-5-en (DBN), 1,8-Diazabi cyclo-[5,4,0]-undec-7-en (DBU) und 1,4-Diazabicyclo-[2,2,2]-octan (DABCO). Man kann auch die Amine der Formel (III) im Überschuß einsetzen und den Amin-Überschuß als Säurebinder wirken lassen.

Die Reaktionstemperaturen können bei dem erfindungsgemäßen Verfahren in einem größeren Bereich variiert werden. Im allgemeinen arbeitet man bei Temperaturen zwischen 0 °C und 100 °C, vorzugsweise bei Temperaturen zwischen 20 °C und 80 °C. Bei reaktionsfähigen Aminen ist Erwärmen meistens kaum erforderlich, weil die Umsetzung schon freiwillig abläuft. Wenn man reaktionsträge Amine bei der erfindungsgemäßen Umsetzung anwendet, kann mehrstündiges Erhitzen erforderlich sein.

Die Umsetzungen werden im allgemeinen unter Normaldruck durchgeführt. Bei leicht flüchtigen und reaktionsträgen Aminen kann es aber vorteilhaft sein, die Umsetzung in geschlossenen Gefäßen und unter erhöhtem Druck vorzunehmen.

Bei der Durchführung des erfindungsgemäßen Verfahrens setzt man auf 1 Rest -CO-Hal im Benzoylhalogenid der Formel (II) 1,00 bis 1,6 Mol, vorzugsweise 1,05 bis 1,3 Mol Amin der Formel (III) und 1 bis 3 Mol, vorzugsweise 1 bis 2 Mol Säureakzeptor oder ein weiteres Mol des Amins der Formel (III) ein. Die Aufarbeitung des Reaktionsgemisches erfolgt nach üblichen Methoden. Die Verbindungen der Formel (I) fallen meistens in fester Form an oder lassen sich - gegebenenfalls nach Einengen der Reaktionslösungen - durch Wasserzugabe abscheiden.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber,
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophage z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodop tera spp., Trichoplusia ni, Carpocapsa pomonella,-Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie de ren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene-und Vorratsschädlinge zeichnen sich die Wirkstoffe durch eine hervorragende Residualwirkung auf Holz und Ton sowie durch eine gute Alkalistabilität auf gekälkten Unterlagen aus.

Wie bereits oben dargelegt, weisen die erfindungsgemäßen neuen Verbindungen der Formel (I) gegenüber entsprechenden vorbekannten Verbindungen in überraschender Weise eine besonders günstige Warmblütertoxizität und gleichzeitig eine sehr gute Wirksamkeit gegen tierische Schädlinge auf. Diese Eigenschaften können in vielen Fällen die Anwendung der Wirkstoffe erheblich erleichtert, insbesondere in Fällen, wo bei der Ausbringung nicht ausreichend erfahrenes Personal zur Verfügung steht und die Einhaltung der bei der Schädlingsbekämpfung erforderlichen und üblichen Vorsichtsmaßnahmen und Sorgfalt nicht voll gewährleistet ist.

Herstellungsbeispiele

Beispiel 1

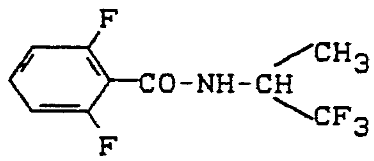

17,65 g (0,1 Mol) 2,6-Difluorbenzoylchlorid, gelöst in 50 ml Toluol, werden bei 20 °C - 30 °C zu einer Lösung von 11,30 g (0,1 Mol) 2-Amino-1,1,1-trifluorpropan und 13,5 g (0,1 Mol) N,N-Dimethylbenzylamin in 200 ml Toluol getropft und bei 50 °C - 60 °C gerührt, wobei N,N-Dimethylbenzylamin-Hydrochlorid ausfällt. Anschließend wird auf 20 °C abgekühlt, mit Wasser extrahiert und die organische Phase im Vakuum eingeengt. Der farblose Rückstand wird aus Waschbenzin und/oder Toluol umkristallisiert.

Man erhält 21,6 g (85,4 % der Theorie) 2,6-Difluorbenzoesäure-N-(1,1,1-trifluor-prop-2-yl)-amid vom Schmelzpunkt 167 °C - 168 °C.

Beispiel 2

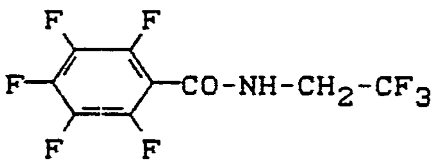

21,4 g (0,1 Mol) 2,3,4,5,6-Pentafluorbenzoylfluorid, gelöst in 50 ml Toluol, werden bei 20 °C - 30 °C zu einer Lösung von 9,9g (0,1 Mol) 2,2,2-Trifluor-1-amino-ethan und 13,5 g (0,1 Mol) N,N-Dimethylbenzylamin in 250 ml Toluol getropft. Anschließend wird bei 50 °C - 60 °C gerührt, auf 20 °C abgekühlt, mit Wasser versetzt und das feste Reaktionsprodukt abgesaugt. Der Rückstand wird mit Wasser gewaschen und getrocknet.

Man erhält 25,2 g (86 % der Theorie) 2,3,4,5,6-Pentafluorbenzoesäure-N-(2,2,2-trifluorethyl)-amid.

Beispiel 3

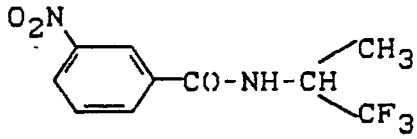

18,55 g (0,01 Mol) 3-Nitrobenzoylchlorid in 50 ml Toluol werden bei 20 °C - 30 °C zu einer Mischung aus 14,95 g (0,1 Mol) 2-Amino-1,1,1-trifluorpropan-Hydrochlorid und 27 g (0,2 Mol) N,N-Dimethylbenzylamin in 250 ml Toluol getropft. Anschließend wird bei 50 °C - 60 °C gerührt. Nach Abkühlen auf 20 °C wird mit Wasser versetzt, und das feste Reaktionsprodukt abgesaugt. Der Rückstand wird mit Wasser gewaschen und getrocknet.

Man erhält 19,8 g (75,6 % der Theorie) 3-Nitro-benzoesäure-N-(1,1,1-trifluor-prop-2-yl)-amid vom Schmelzpunkt 130 °C - 131 °C.

Beispiel 4

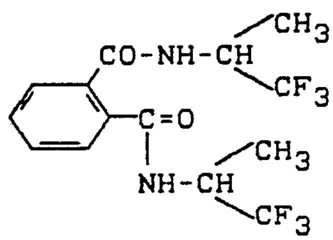

20,3 g (0,1 Mol) o-Phthalylchlorid, gelöst in 100 ml Toluol, werden bei 20 °C - 30 °C zu einer Lösung von 22,6 g (0,2 Mol) 2-Amino-1,1,1-trifluorpropan und 27,0 g (0,2 Mol) N,N-Dimethylbenzylamin in 300 ml Toluol getropft. Anschließend wird bei 50 °C - 60 °C gerührt. Nach Abkühlen auf 20 °C wird mit Wasser versetzt und das feste Reaktionsprodukt abgesaugt. Der Rückstand wird mit Wasser gewaschen und getrocknet.

Man erhält 29,2 g (82 % der Theorie) o-Phthalsäure-bis-[N-(1,1,1-trifluor-prop-2-yl) amid vom Schmelzpunkt 230 °C - 231 °C.

Analog Beispiel 1 bis 4 können die folgenden Verbindungen der Formel (I) hergestellt werden:

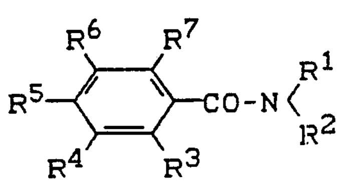

(I)

Tabelle 3

| Beisp.-Nr. | $R^1$ | $R^2$ | $R^3$ | $R^4$ | $R^5$ | $R^6$ | $R^7$ | Fp [°C] |
|---|---|---|---|---|---|---|---|---|
| 5 | H | $-CH_2CF_3$ | F | H | H | H | F | 107 - 109 |
| 6 | $CH_3$ | $-CH_2CF_3$ | F | H | H | H | F | 79 - 81 |
| 7 | H | 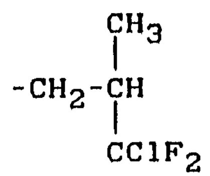 | F | H | H | H | F | 98 - 100 |
| 8 | H | 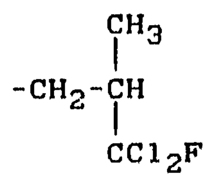 | F | H | H | H | F | 73 - 75 |
| 9 | H | $-CH_2CH_2CF_3$ | F | H | H | H | F | 74 - 76 |
| 10 | H | $-C(CH_3)_2CH_2F$ | F | H | H | H | F | 128 - 130 |
| 11 | H | $-C(CH_3)_2CF_3$ | F | H | H | H | F | 105 - 107 |
| 12 | H | $-CH_2CH_2CF_3$ | H | H | Cl | H | H | 114 - 116 |
| 13 | H | $-CH_2CH_2CF_3$ | H | F | Cl | H | Cl | 88 - 89 |
| 14 | H | $-CH_2CH_2CF_3$ | F | F | F | F | F | 75 - 76 |
| 15 | H | $-CH_2CH_2CF_3$ | H | H | H | $CH_3$ | H | 58 - 60 |
| 16 | H | $-C(CH_3)_2CF_3$ | H | H | H | H | Cl | 82 - 83 |

Tabelle 3 - Fortsetzung

| Beisp.-Nr. | $R^1$ | $R^2$ | $R^3$ | $R^4$ | $R^5$ | $R^6$ | $R^7$ | Fp [°C] |
|---|---|---|---|---|---|---|---|---|
| 17 | H | $-CH_2CH_2CF_3$ | H | H | H | $CF_3$ | H | $n_D^{20}$:1,4465 |
| 18 | H | $-CH(CH_3)CF_3$ | F | F | F | F | F | 150 - 151 |
| 19 | H | $-CH_2CF_3$ | F | Cl | F | Cl | F | 131 - 132 |
| 20 | H | $-CH_2CH_2CF_3$ | F | Cl | F | Cl | F | 118 - 120 |
| 21 | H | $-CH(CH_3)CF_3$ | F | Cl | F | Cl | F | 175 - 177 |
| 22 | H | $-CH_2CF_3$ | H | F | Cl | H | Cl | 126 - 128 |
| 23 | H | $-CH(CH_3)CF_3$ | H | F | Cl | H | Cl | 137 - 139 |
| 24 | H | $-CH_2CF_3$ | H | H | H | H | $CF_3$ | 125 - 127 |
| 25 | H | $-CH(CH_3)CF_3$ | H | H | H | H | $CF_3$ | 118 - 120 |
| 26 | H | $-CH(CH_3)CF_3$ | H | H | H | H | H | 102 - 103 |

<u>Tabelle 3</u> - Fortsetzung

| Beisp.-Nr. | $R^1$ | $R^2$ | $R^3$ | $R^4$ | $R^5$ | $R^6$ | $R^7$ | Fp [° C] |
|---|---|---|---|---|---|---|---|---|
| 27 | H | $-CH\left\langle\begin{matrix}CH_3\\CF_3\end{matrix}\right.$ | H | H | Cl | H | H | 130 - 131 |
| 28 | H | $-CH\left\langle\begin{matrix}CH_3\\CF_3\end{matrix}\right.$ | H | H | $NO_2$ | H | H | 110 - 112 |
| 29 | H | $-CH\left\langle\begin{matrix}CH_3\\CF_3\end{matrix}\right.$ | H | H | H | H | Cl | 128 - 129 |
| 30 | H | $-CH_2CH_2CF_3$ | H | H | $CH_3$ | H | H | 118 - 120 |
| 31 | H | $-CH_2CF_3$ | H | H | $-CONH-CH_2CF_3$ | H | H | >250 |
| 32 | H | $-CH_2CH_2CF_3$ | H | H | $-CONH-CH_2CH_2CF_3$ | H | H | 211 - 212 |
| 33 | H | 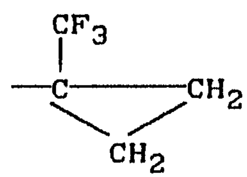 | F | H | H | H | F | 174 - 175 |
| 34 | H | $-CH(CH_2F)_2$ | F | H | H | H | F | 122 - 124 |
| 35 | H | $-CH\left\langle\begin{matrix}CH_3\\CF_3\end{matrix}\right.$ | H | H | H | $CF_3$ | H | 77 - 78 |

<u>Tabelle 3</u> - Fortsetzung

| Beisp.-Nr. | $R^1$ | $R^2$ | $R^3$ | $R^4$ | $R^5$ | $R^6$ | $R^7$ | Fp [° C] |
|---|---|---|---|---|---|---|---|---|
| 36 | H | 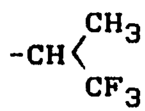 | H | H | H | Cl | H | 94 - 95 |
| 37 | H | 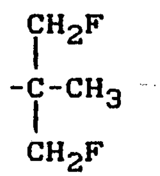 | F | H | H | H | F | 119 - 120 |
| 38 | H | $-C(CH_3)_2CH_2F$ | H | F | Cl | H | Cl | 80 - 81 |
| 39 | H | 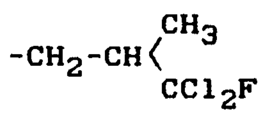 | H | F | Cl | H | Cl | 76 - 78 |
| 40 | H | 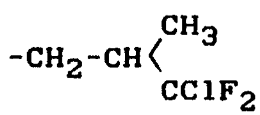 | H | H | H | H | Cl | 98 - 99 |
| 41 | H | 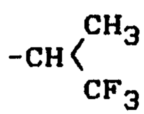 | F | H | H | H | F | 177 - 178 |

<u>Tabelle 3</u> - Fortsetzung

| Beisp.-Nr. | $R^1$ | $R^2$ | $R^3$ | $R^4$ | $R^5$ | $R^6$ | $R^7$ | Fp [°C] |
|---|---|---|---|---|---|---|---|---|
| 42 | H | $-CH\langle^{CH_3}_{CF_3}$ | H | H | H | H | F | 82 |
| 43 | H | $-C(CH_3)_2CF_3$ | H | H | H | H | F | $n_D^{20}$ : 1,4674 |
| 44 | H | $-CH_2CH_2CF_3$ | H | H | H | H | F | 67 |
| 45 | H | $-CH_2CF_3$ | H | H | H | H | F | 88 |
| 46 | $CH_3$ | $-CH_2CF_3$ | H | H | H | H | F | $n_D^{20}$ : 1,4655 |
| 47 | $CH_3$ | $-CH_2CF_3$ | H | H | $CF_3$ | H | H | $n_D^{20}$ : 1,4438 |
| 48 | H | $-CH_2CF_3$ | H | H | $CF_3$ | H | H | 116 |
| 49 | H | $-CH_2CH_2CF_3$ | H | H | $CF_3$ | H | H | 108 |

Tabelle 3 - Fortsetzung

| Beisp.-Nr. | $R^1$ | $R^2$ | $R^3$ | $R^4$ | $R^5$ | $R^6$ | $R^7$ | Fp [°C] |
|---|---|---|---|---|---|---|---|---|
| 50 | H | $-CH(CH_3)CF_3$ | H | H | $CF_3$ | H | H | 131 |
| 51 | $CH_3$ | $-CH_2CF_3$ | F | F | $CF_3$ | F | F | $n_D^{20}$: 1,4199 |
| 52 | H | $-CH_2CF_3$ | F | F | $CF_3$ | F | F | 118 |
| 53 | H | $-CH_2CH_2CF_3$ | F | F | $CF_3$ | F | F | 118 |
| 54 | H | $-CH(CH_3)CF_3$ | F | F | $CF_3$ | F | F | 124 |

Ausgangsverbindungen der Formel (III) und (IIIa)

Beispiel (III-1)

$H_2N-CH(CH_2F)_2$

45 g (0,2 Mol) N-(1,3-Difluor-2-propyl)-phthalimid werden in 100 ml Wasser suspendiert und nach Zugabe von 200 ml konz. Salzsäure während 5 Stunden zum Rückfluß erhitzt. Der nach dem Erkalten ausgefallene Feststoff (Phthalsäure) wird abfiltriert, das Filtrat mit 20 %iger wäßriger Natronlauge extrahiert und die Extrakte über Magnesiumsulfat getrocknet. Nach Abdestillieren des Lösungsmittels bei Normaldruck wird der Rückstand im Wasserstrahlvakuum fraktioniert.

Man erhält 13,5 g (71 % der Theorie) 2-(1,3-Difluor)-propylamin vom Kp. 30 - 34 °C/50 mbar und dem Brechungsindex $n_D^{20}$ = 1,3802.

Beispiel (IIIa-1)

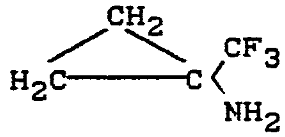 x HCl

40 g (0,4 Mol) 1-Amino-cyclopropancarbonsäure werden mit 100 g $SF_4$ und 50 ml HF 8 Stunden lang in einem $V_4A$-Rührau toklaven bei 120 °C unter Eigendruck (25 - 30 bar) umgesetzt. Nach Abdestillieren der flüchtigen Bestandteile wird mit 45 %iger Natronlauge alkalisch gestellt und das Produkt anschließend durch Wasserdampfdestillation abgetrennt. Das Wasserdampfdestillat wird mit konz. Salzsäure sauer gestellt.

Nach Einengen und Trocknung erhält man 40 g (62 % der Theorie) 1-Trifluormethyl-cyclopropylamin-Hydrochlorid mit einem Schmelzpunkt >260 °C.

Beispiel (IIIa-2)

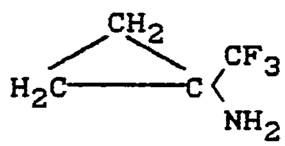

Zu 32,4 g (0,2 Mol) 1-Trifluormethylcyclopropylamin-Hydrochlorid aus Beispiel (IIIa-1) werden 44 g (0,22 Mol) 20 %ige wäßrige Natronlauge bei 80 °C - 90 °C Ölbadtemperatur in ca. 15 min. getropft. Gleichzeitig wird das freigesetzte Amin abdestilliert. Nach Trocknen über Magnesiumsulfat wird redestilliert.

Man erhält 22 g (89 % der Theorie) 1-Trifluormethylcyclopropylamin mit einem Brechungsindex $n_D^{20}$ = 1,3483.

Die günstige Warmblütertoxizität der neuen Verbindungen der Formel (I) kann durch die folgenden Vergleichswerte demonstriert werden:

Beispiel A

$LD_{50}$-Werte an Ratten (oral)

| Wirkstoff | $LD_{50}$ (mg Wirkstoff/kg Körpergewicht) |
|---|---|
| 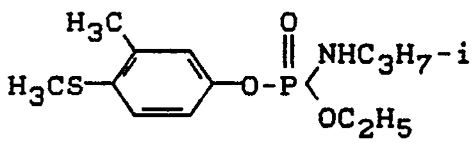<br>Fenamiphos (common name)<br>Stand der Technik (A) | 10 - 20 |
| 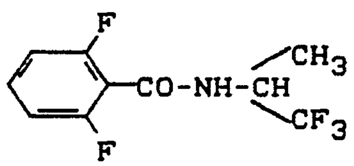<br>(1) | > 1000 |

Die Werte wurden in üblicher Weise ermittelt. Die Wirkstoffe wurden in Polyethylenglykol LUTROL (Warenzeichen der BASF AG, Ludwigshafen, Bundesrepublik Deutschland) gelöst und die Lösung den Ratten mit der Schlundsonde verabreicht.

Beispiel B

Grenzkonzentrations-Test

Testnematode: Globodera rostochiensis
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 18 °C.

Nach sechs Wochen werden die Kartoffelwurzeln auf Zysten untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. bei Wirkstoffkonzentrationen von 2,5, 5 bzw. 10 ppm die erfindungsgemäßen Verbindungen der Herstellungsbeispiele (1), (5), (29) und (33) sowie die bekannte Verbindung (A) einen Wirkungsgrad von 95 %.

Beispiel C

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27 °C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen bei Wirkstoffkonzentration von 1,25 ppm - 10 ppm die erfindungsgemäßen Verbindungen (1), (6), (29) und (33) und die bekannte Verbindung (A) einen Wirkungsgrad von 95 %.

## Ansprüche

1. N-substituierte Benzamide der allgemeinen Formel (I)

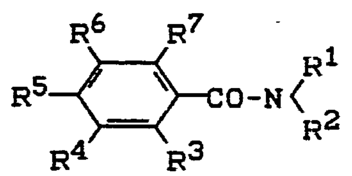

(I)

in welcher
$R^1$ für Wasserstoff oder Alkyl steht,
$R^2$ für Halogenalkyl mit mehr als einem Kohlenstoffatom oder für durch Halogen oder Halogenalkyl substituiertes Cycloalkyl steht,
$R^3$, $R^4$, $R^5$, $R^6$ und $R^7$ gleich oder verschieden sind und für Wasserstoff, Halogen, Nitro, Alkyl, Halogenalkyl, Halogenalkoxy, Halogenalkylthio oder für den Rest -CONR$^1$R$^2$ stehen.

2. N-substituierte Benzamide gemäß Anspruch 1, dadurch gekennzeichnet, daß in der Formel (I)
$R^1$ für Wasserstoff oder Alkyl mit 1 bis 6 Kohlenstoffatomen steht,
$R^2$ für Halogenalkyl mit 2 bis 8 Kohlenstoffatomen und 1 bis 12 Halogenatomen wie Fluor und/oder Chlor, sowie für durch Halogen wie Fluor und/oder Chlor oder Halogen-$C_1$-$C_2$-alkyl substituiertes Cycloalkyl mit 3 bis 8 Kohlenstoffatomen steht,
$R^3$, $R^4$, $R^5$, $R^6$ und $R^7$ gleich oder verschieden sind und für Wasserstoff, Fluor, Chlor, Brom, Nitro, Alkyl mit 1 bis 4 Kohlenstoffatomen, Halogenalkyl, Halogenalkoxy oder Halogenalkylthio mit jeweils 1 bis 4 Kohlenstoffatomen im Alkylteil und 1 bis 6 Halogenatomen sowie für den Rest -CONR$^1$R$^2$ stehen.

3. N-substituierte Benzamide gemäß Ansprüchen 1 und 2, dadurch gekennzeichnet, daß in der allgemeinen Formel (I)
$R^1$ für Wasserstoff oder Alkyl mit 1 bis 4 Kohlenstoffatomen steht,
$R^2$ für Halogenalkyl mit 2 bis 6 Kohlenstoffatomen und 1 bis 8 Fluor-oder 1 bis 8 Fluor-und Chloratomen sowie für durch Fluor, Chlor und/oder Trifluormethyl substituiertes Cycloalkyl mit 3 bis 6 Kohlenstoffatomen steht,
$R^3$, $R^4$, $R^5$, $R^6$ und $R^7$ gleich oder verschieden sind und für Wasserstoff, Fluor, Chlor, Brom, Nitro, Alkyl mit 1 oder 2 Kohlenstoffatomen, Halogenalkyl, Halogenalkoxy oder Halogenalkylthio mit jeweils 1 oder 2 Kohlenstoffatomen im Alkylteil und 1 bis 4 Fluor-und/oder Chloratomen sowie für den Rest -CONR$^1$R$^2$ stehen.

4. N-substituierte Benzamide gemäß Ansprüchen 1 bis 3, dadurch gekennzeichnet, daß in der allgemeinen Formel (I)
$R^1$ für Wasserstoff, Methyl, Ethyl, n-Propyl, i-Propyl oder n-Butyl steht,
$R^2$ für 2-Fluorethyl, 2,2-Difluorethyl, 2,2,2-Trifluorethyl, 3-Fluor-prop-1-yl, 3,3-Difluor-prop-1-yl, 3,3,3-Trifluor-prop-1-yl, 3-Fluor-prop-2-yl, 3,3-Difluor-prop-2-yl, 3,3,3-Trifluor-prop-2-yl, 2-Fluor-2-methyl-prop-2-yl, 2,2-Difluor-2-methyl-prop-2-yl, 2-Methyl-2,2,2-trifluor-prop-2-yl, 2-Methyl-3,3,3-trifluor-prop-1-yl, 2-Methyl-3,3,3-chlordifluor-prop-1-yl, 2-Methyl-3,3,3-dichlorfluor-prop-1-yl, 1,3-Difluor-prop-2-yl, 1,1,1,3,3,3-Hexafluor-prop-2-yl, 3-Fluor-2-fluormethyl-prop-2-yl, 1,3-Difluor-2-fluor-methyl-prop-2-yl, 1-Trifluormethyl-cycloprop-1-yl und 2,2-Difluor-cycloprop-1-yl steht und
$R^3$, $R^4$, $R^5$, $R^6$ und $R^7$ gleich oder verschieden sind und für Wasserstoff, Fluor, Chlor, Brom, Nitro, Methyl, Trifluormethyl, Trifluormethoxy, Trifluormethylthio sowie für den Rest -CONR$^1$R$^2$ stehen.

5. Verfahren zur Herstellung von N-substituierten Benzamiden der allgemeinen Formel (I)

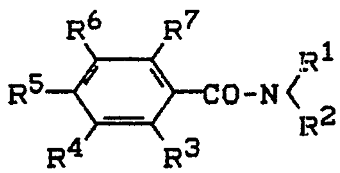

(I)

in welcher
$R^1$ für Wasserstoff oder Alkyl steht,
$R^2$ für Halogenalkyl mit mehr als einem Kohlenstoffatom oder für durch Halogen oder Halogenalkyl substituiertes Cycloalkyl steht,
$R^3$, $R^4$, $R^5$, $R^6$ und $R^7$ gleich oder verschieden sind und für Wasserstoff, Halogen, Nitro, Alkyl, Halogenalkyl, Halogenalkoxy, Halogenalkylthio oder für den Rest -CONR$^1$R$^2$ stehen,
dadurch gekennzeichnet, daß man Benzoylhalogenide der Formel (II)

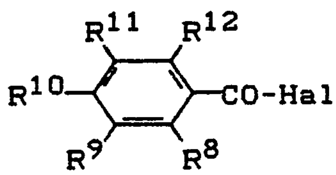

(II)

in welcher
$R^8$, $R^9$, $R^{10}$, $R^{11}$ und $R^{12}$ gleich oder verschieden sind und für Wasserstoff, Halogen, Nitro, Alkyl, Halogenalkyl, Halogenalkoxy, Halogenalkylthio oder für den Rest -CO-Hal stehen und
Hal für Halogen steht,
mit Aminen der Formel (III)
$HNR^1R^2$ (III)
in welcher
$R^1$ und $R^2$ die oben angegebenen Bedeutungen haben,
oder mit den entsprechenden Hydrohalogeniden, gegebenenfalls in Gegenwart von Säureakzeptoren und gegebenenfalls in Gegenwart von Verdünnungsmitteln umsetzt.

6. Schädlingsbekämpfungsmittel, gekennzeichnet durch einen Gehalt an mindestens einem N-substituierten Benzamid der Formel (I).

7. Insektizide und nematizide Mittel, gekennzeichnet durch einen Gehalt an mindestens einem N-substituierten Benzamid der Formel (I).

8. Verfahren zur Bekämpfung von Insekten und/oder Nematoden, dadurch gekennzeichnet, daß man N-substituierte Benzamide der Formel (I) auf Insekten und/oder Nematoden und/oder deren Lebensraum einwirken läßt.

9. Verwendung von N-substituierten Benzamiden der Formel (I) zur Bekämpfung von tierischen Schädlingen, insbesondere von Insekten und Nematoden.

10. Verfahren zur Herstellung von Schädlingsbekämpfungsmitteln, dadurch gekennzeichnet, daß man N-substituierte Benzamide der Formel (I) mit Streckmitteln und/oder oberflächenaktiven Mitteln vermischt.